(19) Europäisches Patentamt
European Patent Office
Office européen des brevets

(11) Veröffentlichungsnummer: **0 339 383 A2**

(12) # EUROPÄISCHE PATENTANMELDUNG

(21) Anmeldenummer: **89106602.9**

(22) Anmeldetag: **13.04.89**

(51) Int. Cl.⁴: **C07C 101/453 , C07C 97/00 , C07C 121/66 , C07C 69/738 , B41M 5/26 , B41M 5/12**

(30) Priorität: **23.04.88 DE 3813807**

(43) Veröffentlichungstag der Anmeldung: **02.11.89 Patentblatt 89/44**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Mayer, Udo, Dr.**
**Max-Slevogt-Strasse 27**
**D-6710 Frankenthal(DE)**
Erfinder: **Güntner, Andreas, Dr.**
**Schubertstrasse 4**
**D-6804 Ilvesheim(DE)**

(54) **Bisstyrylmethane.**

(57) Bisstyrylmethane der Formel (I)

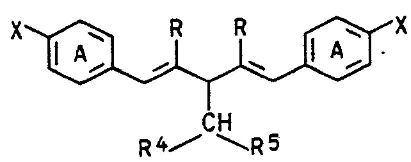

(I) ,

in der R für H oder Methyl oder beide R für gegebenenfalls durch Alkyl substituiertes Di- oder Trimethylen, X für

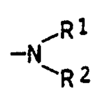

oder $-OR^3$, worin
$R^1$ und $R^2$ gegebenenfalls substituiertes Alkyl oder $R^1$ gegebenenfalls substituiertes Phenyl oder

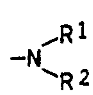

einen gesättigten fünf- oder sechsgliedrigen Heterocyclus und
wobei $R^1$ und/oder $R^2$ mit den orthoständigem C-Atom im Ring A auch einen Fünf- oder Sechsring bilden können und
$R^3$ Methyl oder Ethyl bedeuten, und $R^4$ und $R^5$ für -CN, $-COCH_3$,

EP 0 339 383 A2

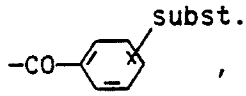

,

-CO-NH-$R^7$ oder -COO$R^7$ stehen, worin $R^7$ Alkyl bedeuten oder

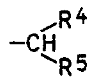

für einen carboncyclischen oder heterocyclischen Fünf- oder Sechsring stehen und die Ringe A gegebenenfalls durch $C_1$-$C_4$-Alkyl oder $C_1$-$C_4$-Alkoxy substituiert sind.
(I) eignet sich hervorragend als Farbbildner in druck- und wärmeempfindlichen Aufzeichungssystemen.

## Bisstyrylmethane

Aus der JP-A-23 766/1985 sind Bisstyrylmethane der Formel (II)

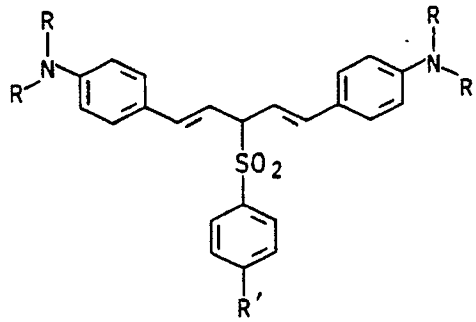

(II) ,

bekannt, in der

R für niederes Alkyl und

R′ für Wasserstoff oder niederes Alkyl stehen. Die Verbindungen (II) sind für die Anwendung in wärme- und druckempfindlichen Aufzeichnungssystemen geeignet.

Aufgabe der vorliegenden Erfindung war es, weitere für druck- und wärmeempfindliche Aufzeichnungssysteme als Farbbildner geeignete Verbindungen bereitzustellen.

Gegenstand der Erfindung sind Bisstyrylmethane der allgemeinen Formel (I)

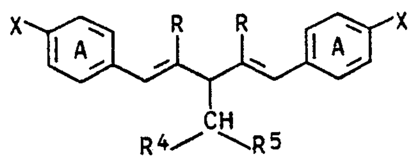

(I) ,

in der

R unabhängig voneinander für Wasserstoff oder $C_1$-$C_4$-Alkyl oder beide R zusammen für eine gegebenenfalls durch $C_1$- bis $C_4$-Alkyl ein- bis dreifach substituierte Di- oder Trimethylenbrücke,

X unabhängig voneinander für

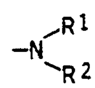

oder -O-$R^3$, worin

$R^1$ und $R^2$ unabhängig voneinander Wasserstoff, gegebenenfalls durch Hydroxy, Cyan, Phenyl, Fluor, Chlor oder Brom substituiertes $C_1$-bis $C_6$-Alkyl oder $R^1$ auch gegebenenfalls durch $C_1$- bis $C_4$-Alkyl, $C_1$- bis $C_4$-Alkoxy, Chlor, Fluor oder Brom substituiertes Phenyl oder

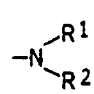

einen gesättigten heterocyclischen fünf- oder sechsgliedrigen Ring und wobei

$R^1$und/oder $R^2$ mit dem orthoständigen C-Atom im Ring A einen Fünf- oder Sechsring bilden können.

$R^3$ $C_1$-$C_{12}$-Alkyl oder Benzyl bedeuten, und

$R^4$ und $R^5$ unabhängig voneinander für -CN, -$COCH_3$,

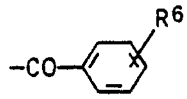 ,

-CONH-$R^7$ oder -COOR$^7$ stehen, worin

$R^6$ Wasserstoff, $C_1$- bis $C_4$-Alkyl, Chlor, Brom oder Fluor und

$R^7$ $C_1$- bis $C_8$-Alkyl bedeuten oder

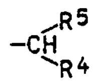

für einen carbocyclischen oder heterocyclischen Fünf- oder Sechsring stehen und wobei die Ringe A gegebenenfalls durch $C_1$-$C_4$-Alkyl oder $C_1$- bis $C_4$-Alkoxy substituiert sind.

Die erfindungsgemäßen Verbindungen (I) liefern bei Einwirkung von Säuren bzw. sauren Materialen im NIR- und VIS-Bereich sehr intensive Färbungen, die deutlich intensiver sind als die der Farbbildner des Standes der Technik vom Typ (II). Außerdem färben die Farbbildner (I) unbehandeltes Papier weniger als die Verbindungen (II).

In der Formel (I) steht R z.B. für Methyl, Ethyl, Propyl, Isopropyl, Butyl Isobutyl oder Wasserstoff oder beide R zusammen für einen gegebenenfalls durch Alkyl substituierte Di- oder Trimethylengruppe, wie Trimethylen-1,3, 3-Methyltrimethylen-1,3, 1,1,3-Trimethyltrimethylen-1,3, 2-tert-Butyltrimethylen-1,3.

Vorzugsweise ist R Wasserstoff.

In

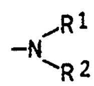

stehen

$R^1$ und $R^2$ unabhängig voneinander für Wasserstoff, für gegebenenfalls durch Hydroxy, Cyan, Phenyl, Fluor, Chlor oder Brom substituiertes $C_1$-bis $C_6$-Alkyl, wobei wenn beide X für

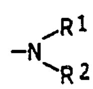

stehen, diese gleich oder verschieden sein können

$R^1$ kann außerdem für Phenyl oder für durch $C_1$-$C_4$-Alkyl, $C_1$- bis $C_4$- Alkoxy, Chlor, Brom oder Fluor substituiertes Phenyl stehen. Im einzelnen sind für $R^1$ und $R^2$ z.B. zu nennen: Methyl, Ethyl, n-Propyl, n- und i-Butyl, Pentyl, Hexyl, Cyclohexyl, Benzyl, 2-Phenylethyl, 2-Chlorethyl, 2-Cyanethyl und 2-Hydroxiethyl.

Als gegebenenfalls substituiertes Phenyl kommen für $R^1$ z.B. in Betracht: 4-Methoxiphenyl, 4-Ethoxiphenyl, 4-Chlorphenyl.

Für

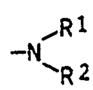

sind als gesättigte 5- oder 6-gliedrige heterocyclische Reste z.B. solche zu nennen, die sich vom Piperidin, Morpholin, Piperazin, $N'$-$C_1$- bis $C_4$-Alkylpiperazin und Pyrrolidin ableiten.

Bevorzugt sind Verbindungen (I), bei denen X für

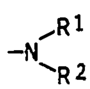

steht. Von diesen sind solche besonders hervorzuheben, bei denen $R^1$ und $R^2$ = $C_1$- bis $C_4$-Alkyl sind.

$R^5$ und $R^4$ stehen für

$-CN$ , 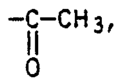 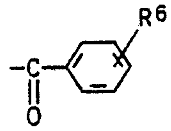 , 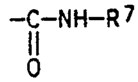 oder 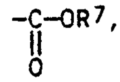, worin

$R^7$ $C_1$- bis $C_8$-Alkyl, vorzugsweise $C_1$- bis $C_4$-Alkyl und

$R^6$ H, $C_1$- bis $C_4$-Alkyl, Chlor, Brom oder Fluor sind.

$R^5$ und $R^4$ stehen vorzugsweise für

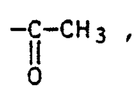 , 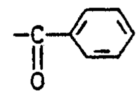 oder 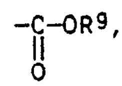, worin

$R^9$ $C_1$- bis $C_4$-Alkyl ist.

Als Alkyl kommen für $R^7$ z.B. in Betracht: Methyl, Ethyl, n- und i-Propyl, n- und i-Butyl, Butyl-2, Pentyl, i-Pentyl, Hexyl, i-Hexyl, Heptyl, n- und i-Octyl, 2-Ethylhexyl-1, von denen die Alkylreste mit 1 bis 4-C-Atomen bevorzugt sind.

Für $R^6$ kommen als Alkyl z.B. die für $R^7$ genannten Alkylreste mit 1-bis 4-C-Atomen in Betracht.

Besonders bevorzugt sind Verbindungen der Formel (I a)

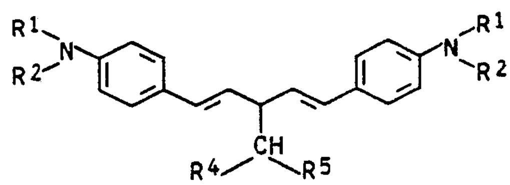

(I a) ,

in der

$R^1$ = $R^2$ $C_1$- bis $C_4$-Alkyl, insbesondere Ethyl oder Methyl bedeuten

und

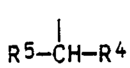 für 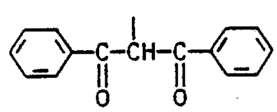 , 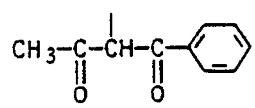 , 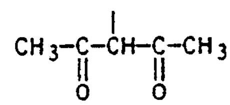

oder 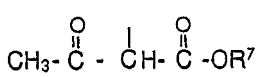 stehen, worin

$R^7$ $C_1$- bis $C_4$-Alkyl, insbesondere Methyl oder Ethyl bedeutet.

Die erfindungsgemäßen Bisstyrylmethane (I) werden vorteilhaft erhalten, wenn man Carbeniumsalze der Formel (III)

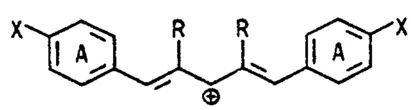

(III) ,

in der

R, X und A die obengenannte Bedeutung besitzen und X⊖ ein Anion bedeutet,mit einer Verbindung der Formel (IV)

$R^5-CH_2R^4$ (IV),

in der

$R^5$ und $R^4$ die obengenannte Bedeutung haben, im Molverhältnis 1 : 1,2 bis 1 : 2 in einer inerten organischen Flüssigkeit in Gegenwart einer Base bei 20 bis 120 °C, vorzugsweise bei 40 bis 80 °C umsetzt.

Geeignete Anionen X⊖ sind z. B. Trichlorozinkat, Tetrachloroferrat-(III), Perchlorat, Tetrafluoroborat, Hydrogensulfat, Nitrat oder Halogenid, wie Chlorid oder Bromid. Für X⊖ sind Trichlorozinkat und Tetrachloroferrat-(III) besonders bevorzugt.

Als inerte organische Flüssigkeiten verwendet man zweckmäßig Alkohole, z.B. Methanol, Ethanol, Propanol, Isopropanol, Butanol oder Isobutanol. Gegebenenfalls werden auch Mischungen dieser Alkohole mit aromatischen Kohlenwasserstoffen, wie Toluol oder Xylol als Reaktionsmedium verwendet.

Geeignete Basen für die Umsetzung des Farbsalzes (III) mit der Verbindung (IV) sind beispielsweise Alkalimetall- oder Erdalkalimetallcarbonate, wie Natriumcarbonat, Kaliumcarbonat, Magnesiumcarbonat oder Calciumcarbonat→ Erdalkalimetalloxide, wie Magnesiumoxid oder Calciumoxid→ oder Alkalimetallalkanolate, wie Natrium- oder Kaliummethanolat, -ethanolat oder -butanolat. Im allgemeinen gibt man 1 bis 3 Mol Base je Mol (III) zu.

Die als Ausgangsstoffe benötigten Farbsalze (III) und Verbindungen (IV) sind bekannt.

Die erfindungsgemäßen Bis(styryl)methane sind schwach farbige bis farblose Verbindungen, deren Lösungen in inerten organischen Lösungsmitteln im Kontakt mit Elektronenacceptoren, Färbungen in blauen Farbtönen geben. Beispiele für Elektronenacceptorsubstanzen sind Carbon- oder Mineralsäuren, Kaolin, Bentonit, aktivierter Ton, Aluminiumsilikat, Attapulgit oder jeder beliebige Ton, sauer reagierende polymere Materialien wie Kondensationsprodukte auf der Basis von Phenolen und/oder Phenolsulfonsäuren, ferner Metalloxide oder -salze, wie Zinkoxid, Aluminiumoxid, Zinkchlorid, Eisenstearat oder Cobaltnaphthenat.

Aufgrund dieser Eigenschaften sind die neuen Verbindungen der Formel (I) als Farbbildner zur Verwendung in druck- und wärmeempfindlichen Aufzeichnungsmaterialien geeignet.

Für die Anwendung in druckempfindlichen Systemen werden die neuen Bis(styryl)methane vorteilhaft in Form ihrer Lösungen in organischen Lösungsmitteln, z.B. Chlorparaffine, partiell hydriertes Di- oder Terphenyl, Alkylbenzole, Alkylnaphthaline, alkylierte Dibenzylbenzole, Paraffinöl, Mineralöl oder auch tiefer siedende Lösungsmittel wie Xylol oder Toluol in Mikrokapseln eingeschlossen und mit diesen der Träger, z.B. Papier beschichtet. Bei Druck tritt dann im Kontakt mit Elektronenacceptoren an der Druckstelle Farbbildung ein.

Geeignete Verfahren zur Herstellung der Mikrokapseln sind z. B. aus der US-A-2 800 457, US-A-2 800 458, DE-A-2 119 933 und der EP-A-26 914 bekannt. Man kann die erfindungsgemäßen Verbindungen auch nach dem in der US-A-3 103 404 beschriebenen Verfahren in Wachs oder Öl-Wachsmischungen fein verteilen und mit diesen Mischungen Träger, wie Folien oder Papier beschichten. Man erhält druckempfindliche Materialien, die zum Durchschreiben auf mit Elektronenacceptorsubstanzen beschichteten Papieren geeignet sind und die nach Gebrauch wie Kohlepapier entfernt werden.

Die erfindungsgemäßen Bis(styryl)methane können auch als Farbbildner in wärmeempfindlichen Aufzeichnungsmaterialien verwendet werden, die auf einem Träger ein Bindemittel, einen Farbbildner und eine Elektronenacceptorsubstanz enthalten. Der Aufbau solcher wärmeempfindlicher Aufzeichnungsmaterialien und die Zusammensetzung der durch Einwirkung von Wärme die Farbe erzeugenden Schichten sind bekannt (z. B. DE-A-2 228 581, DE-A-2 110 854), ebenso die Verfahren und Vorrichtungen, mit denen die Farbbildung erreicht wird.

Die folgenden Beispiele sollen die Erfindung näher erläutern.

Beispiel A

Die zur Herstellung von (I) benötigten Carbeniumsalze wurden nach der folgenden allgemeinen Vorschrift hergestellt.

64 g Bis(p-dimethylaminostyryl)keton wurden in 500 ml Diethylenglykoldimethylether verrührt. Zu dieser Lösung wurden bei 20 -30 °C 18 g Natriumborhydrid hinzugegeben. Der Ansatz wurde 3 Stunden bei Raumtemperatur gehalten und dann eine Stunde auf 70 °C erwärmt. Nach dem Abkühlen wurde der gesamte Inhalt auf eine Lösung aus
800 g Eiswasser,
90 ml Konz. Salzsäure und
60 ml 70 %iger Zinkchloridlösung gegossen und das Gemisch 30 Minuten lang gerührt.

Das ausgefallene Salz wurde abgesaugt.

Aus 64 g Bis-(p-dimethylaminostyryl)-keton wurden 60 g Bis(4-dimethylaminostyryl)-carbeniumtrichlorzinkat der Formel

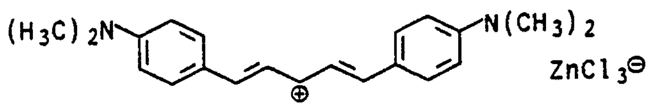

(III.1)

erhalten.
Schmp.: 270 - 274 °C

Beispiel 1

14 g Bis(4-dimethylaminostyryl)carbeniumtrichlorzinkat (III.1), 7 g Natriumcarbonat und 6 g Acetessigsäureethylester wurden in 150 ml Methanol 3 Stunden auf 40 bis 50 °C erwärmt. Nach dieser Zeit wurde das Reaktionsgemisch in 200 ml Toluol und 200 ml Wasser gegeben und verrührt. Die organische Phase wurde abgetrennt, mit Tierkohle gereinigt und eingedampft. Ausbeute: 10 g der Verbindung der Formel

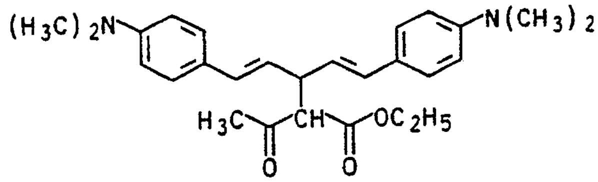

in Form eines farblosen Öls.
Die Lösung in Toluol gibt bei Auftropfen auf CF-Papier eine bläuliche Färbung mit Absorption im NIR-Bereich.
$\lambda_{max}$ (Essigsäure) = 790 nm
IR (KBR): 1735, 1705 $an^{-1}$

Beispiele 2 bis 7

Analog den Angaben des Beispiels 1 wurden die in der folgenden Tabelle angegebenen Farbbildner der Formel (I b) hergestellt.

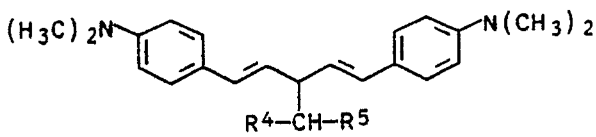

(I b)

| Bsp.-Nr. | $R^4-CH-R^5$ | Schmp. [°C] | Ausbeute | IR (KBR) $cm^{-1}$ | λmax[1] nm |
|---|---|---|---|---|---|
| 2 | NC-CH-CN | 114 - 118 | 36 % | 2200 (-CN) | 790 |
| 3 | $HC_3-CO-CH-CO-CH_3$ | 134 - 136 | 42 % | 1722 $(-\overset{\overset{O}{\|}}{C}-)$ | 790 |
| 4 | $H_5C_6-CO-CH-CO-CH_3$ | öl | 71,5 % | 1710 $(-\overset{\overset{O}{\|}}{C}-)$ | 790 |
| 5 | $HC(CO_2C_2H_5)_2$ | öl | 93 % | 1740 $(-\overset{\overset{O}{\|}}{C}OR)$ | 790 |
| 6 | $NC-HC-CO_2C_2H_5$ | öl | 64 % | 2245 (CN), 1740 | 790 |
| 7 | $H_5C_6-CO-CH-CO-C_6H_5$ | 166 - 170 | 69,4 % | 1685 $(-\overset{\overset{O}{\|}}{C}-)$ | 790 |

1) in Essigsäure

Analog Beispiel 1 wurden die Verbindungen der Formel (I c) hergestellt. Die Bedeutung von X und R sind in der Tabelle angegeben.

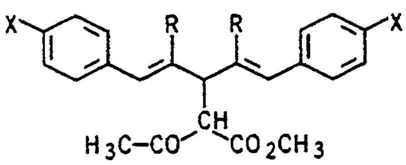

(I c)

| Bsp.-Nr. | X | R | R | Farbe |
|---|---|---|---|---|
| 8 | $-N(C_2H_5)_2$ | H | H | blaugrün |
| 9 | $-N(C_4H_9)_2$ | H | H | blaugrün |
| 10 | $-N(C_2H_5)(CH_2-C_6H_5)$ | H | H | blaugrün |
| 11 | $-N(CH_3)_2$ | $-CH_3$ | $-CH_3$ | blaugrün |
| 12 | $-N(CH_3)_2$ | $-CH_2-CH_2-CH_2-$ | | blaugrün |
| 13 | $-N(CH_3)_2$ | $-CH_2-CH_2-$ | | blaugrün |
| 14 | $-OCH_3$ | H | H | violett |
| 15 | $-O-CH_2-C_6H_5$ | H | H | violett |

**Ansprüche**

1. Bisstyrylmethane der allgemeinen Formel (I)

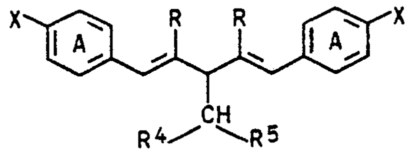

(I) ,

in der

R unabhängig voneinander für Wasserstoff oder $C_1$-$C_4$-Alkyl oder beide R zusammen für eine gegebenenfalls durch $C_1$- bis $C_4$-Alkyl ein- bis dreifach substituierte Di- oder Trimethylenbrücke,

X unabhängig voneinander für

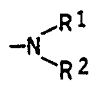

oder -O-$R^3$, worin

$R^1$ und $R^2$ unabhängig voneinander Wasserstoff, gegebenenfalls durch Hydroxy, Cyan, Phenyl, Fluor, Chlor oder Brom substituiertes $C_1$- bis bis $C_6$-Alkyl oder $R^1$ auch gegebenenfalls durch $C_1$- bis $C_4$-Alkyl, $C_1$- bis $C_4$-Alkoxy, Chlor, Fluor oder Brom substituiertes Phenyl oder

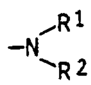

einen gesättigten heterocyclischen fünf- oder sechsgliedrigen Ring und wobei $R^1$ und/oder $R^2$ mit dem orthoständigen C-Atom im Ring A auch einen Fünf- oder Sechsring bilden können und

$R^3$ $C_1$-$C_{12}$-Alkyl oder Benzyl bedeuten, und

$R^4$ und $R^5$ unabhängig voneinander für -CN, $-COCH_3$,

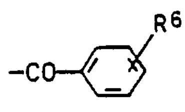

,

-CONH-$R^7$ oder -COO$R^7$, worin

$R^6$ Wasserstoff, $C_1$- bis $C_4$-Alkyl, Chlor, Brom oder Fluor und

$R^7$ $C_1$- bis $C_8$-Alkyl bedeuten, oder

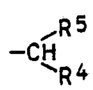

für einen carbocyclischen oder heterocyclischen Fünf- oder Sechsring stehen und wobei die Ringe A gegebenenfalls durch $C_1$-$C_4$-Alkyl oder $C_1$- bis $C_4$-Alkoxy substituiert sind.

2. Bisstyrylmethane gemäß Anspruch 1, dadurch gekennzeichnet, daß X für gleiches oder verschiedenes

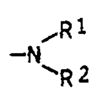

steht.

3. Bisstyrolmethane gemäß Anspruch 1 oder 2, dadurch gekennzeichnet, daß $R^1$ und $R^2$ für $C_1$- bis $C_4$-Alkyl stehen.

4. Bisstyrylmethane gemäß Anspruch 1, 2 oder 3, dadurch gekennzeichnet, daß $R^1$ und $R^2$ gleich sind.

5. Bisstyrylmethane gemäß Anspruch 1, 2, 3 oder 4, dadurch gekennzeichnet, daß

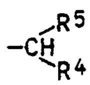 für 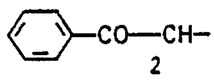 , 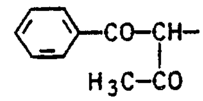 ,

$(H_3C\text{-}CO)_2\text{-}CH$-oder

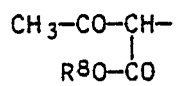

steht, worin

$R^8$ $C_1$- bis $C_4$-Alkyl ist.

6. Bisstyrylmethane gemäß Anspruch 1, gekennzeichnet durch die Formel

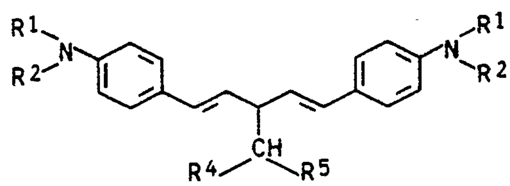 (I a) ,

in der

$R^1$ und $R^2$ für $C_1$-$C_4$-Alkyl und

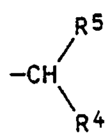 für 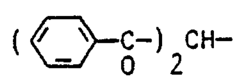 , 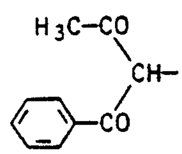 ,

$(H_3CCO\text{-})_2CH$-oder

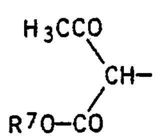

stehen, worin

$R^7$ $C_1$-$C_4$-Alkyl bedeuten.

7. Bisstyrylmethane gemäß Anspruch 6, dadurch gekennzeichnet, daß $R^1 = R^2$ ist.

8. Bisstyrylmethane gemäß Anspruch 6 oder 7, dadurch gekennzeichnet, daß $R^1$ und $R^2$ für Methyl oder Ethyl stehen.

9. Bisstyrylmethane gemäß Anspruch 6, 7 oder 8, dadurch gekennzeichnet, daß $R^7$ Methyl oder Ethyl ist.

10. Verwendung der Bisstyrylmethane gemäß den Ansprüchen 1 bis 9 als Farbbildner in druck- und wärmeempfindlichen Aufzeichnungsmaterialien.